# EUROPEAN PATENT SPECIFICATION

(11) **EP 1 638 414 B1**
(45) Date of publication and mention of the grant of the patent: **17.06.2009**
(21) Application number: 04740058.5
(22) Date of filing: 18.06.2004
(51) Int. Cl.: A23L 1/29, A23L 1/30, A23L 1/305, A61K 35/74, A61K 31/202

(54) **INFANT OR FOLLOW-ON FORMULA**
SÄUGLINGSNÄHRPRÄPARAT ODER FOLGEMILCH
FORMULE INFANTILE

(30) Priority: 23.06.2003 EP 03014056
(43) Date of publication of application: 29.03.2006
(73) Proprietor: Nestec S.A., 1800 Vevey (CH)
(72) Inventor: SECRETIN, Marie-Cristine, CH-1807 Blonay (CH)
(74) Representative: Dixon, Sarah
(86) International application number: PCT/EP2004/006613
(87) International publication number: WO 2004/112507

(56) References cited:
- EP-A- 0 231 904
- EP-A- 0 880 902
- EP-A- 0 904 784
- EP-A- 1 048 226
- WO-A-02/15719
- WO-A-02/060276
- DE-A- 10 219 684
- DE-U- 20 202 562
- US-A- 5 374 657
- US-A1- 2002 004 527
- US-A1- 2003 017 192
- US-A1- 2003 060 509
- US-A1- 2003 077 255
- OGATA T ET AL: "EFFECT OF BIFIDOBACTERIUM LONGUM BB536 YOGHURT ADMINISTRATION ON THE INTESTINAL ENVIRONMENT OF HEALTHY ADULTS" MICROBIAL ECOLOGY IN HEALTH & DISEASE, CHICHESTER, GB, vol. 11, no. 1, March 1999 (1999-03), pages 41-46, XP009023764
- GRILL J P ET AL: "Bifidobacteria and probiotic effects: action of Bifidobacterium species on conjugated bile salts." CURRENT MICROBIOLOGY. JUL 1995, vol. 31, no. 1, July 1995 (1995-07), pages 23-27, XP008036111 ISSN: 0343-8651
- ISOLAURI E ET AL: "Probiotics in the management of atopic eczema." CLINICAL AND EXPERIMENTAL ALLERGY : JOURNAL OF THE BRITISH SOCIETY FOR ALLERGY AND CLINICAL IMMUNOLOGY. NOV 2000, vol. 30, no. 11, November 2000 (2000-11), pages 1604-1610, XP002298117 ISSN: 0954-7894
- SAXELIN M: "LACTOBACILLUS GG - A HUMAN PROBIOTIC STRAIN WITH THOROUGH CLINICAL DOCUMENTATION" FOOD REVIEWS INTERNATIONAL, NEW YORK, NY, US, vol. 13, no. 2, 1997, pages 293-313, XP000199732
- KALLIOMAKI M ET AL: "Probiotics and prevention of atopic disease: 4-year follow-up of a randomised placebo-controlled trial" LANCET THE, LANCET LIMITED. LONDON, GB, vol. 361, no. 9372, 31 May 2003 (2003-05-31), pages 1869-1871, XP004428374 ISSN: 0140-6736
- KANKAANPAA P E ET AL: "The influence of polyunsaturated fatty acids on probiotic growth and adhesion." FEMS MICROBIOLOGY LETTERS 194 (2) 149-153 2001 DEP. OF BIOCHEM. & FOOD CHEM., UNIV. OF TURKU, 20014 TURKU, FINLAND. TEL. +358 (2) 333 6873. FAX +358 (2) 333 6860. E-MAIL PAERKA(A)UTU.FI, 2001, XP002261972
- MATSUMOTO M ET AL: "ADHESIVE PROPERTY OF BIFIDOBACTERIUM LACTIS LKM512 AND PREDOMINANT BACTERIA OF INTESTINAL MICROFLORA TO HUMAN INTESTINAL MUCIN" CURRENT MICROBIOLOGY, NEW YORK, NY, US, vol. 44, no. 3, 2002, pages 212-215, XP008024172 ISSN: 0343-8651

## Description

### Field of the invention

The present invention relates to a new and inventive nutritional composition intended for infants and/or young children, as well as to a method for strengthening natural immune defences and to a method for promoting a healthy mental development in infants or young children by fully or partly feeding them with the afore-mentioned formula

### Background of the invention

The composition of human milk serves as a valuable reference for improving infant formula. However, human milk contains living cells, hormones, active enzymes, immunoglobulins and components with unique molecular structures that cannot be replicated in infant formula. Unlike human milk, infant formula must remain stable on the shelf for up to thirty-six (36) months. These fundamental differences between human milk and infant formula often mandate differences in the composition to achieve similar clinical outcome.

The study of human milk components has stimulated many investigations into what constituents may be added to an improved infant formula. Greater knowledge of the composition of human milk affords the opportunity to design infant formulas that are closer in composition to human milk. However, it becomes increasingly apparent that infant formula can never exactly duplicate human milk. Many constituents in human milk are bioactive and because of synergies among these components, there is little reason to believe that the same compound would have the same bioactivity in infant formula. The likelihood of this possibility is further diminished when the impact of heat treatment for sterilization and long-term storage of the formula is considered.

The composition of human milk differs appreciably from that of other species and much attention has been paid to the various components. Several investigators have reported on the nucleotide content of milk from humans. Numerous publications have also discussed various lipid, oil or fat blends for use in an artificial nutritional for human infants.

There is a need for new formulae, providing to the infant or the young child a nutritional contribution with a unique combination of protective nutrients, especially ensuring growth and metabolic patterns similar to those of breastfed infants, thus resulting in similar health characteristics in later childhood and adulthood.

EP 231904 discloses a new fat mix rich in long chain polyunsaturated fatty acids such as ARA and DHA and the use of such fat mixes in the preparation of infant formulas. US Patent No. 5374657 discloses the use of certain micro-organisms to produce long chain polyunsaturated fatty acids such as ARA and DHA and the incorporation of such microbial LC-PUFA oils in infant formula. US Patent Application No. 2003/0017i92 discloses a process for producing extended shelf-life ready to use nutritional compositions such as infant formulas which contain probiotics. EP 904784 discloses nutritional compositions comprising a minimum of three different probiotic strains with the intention of providing protection against pathogenic infection all the way along the gastro-intestinal tract thus obviating the need to identify the micro-organism responsible for the infection. Kankaanpää et al discuss the influence of polyunsaturated fatty acids on growth and adhesion of certain lactobacilli and note that the growth and adhesion of the probiotic Lactobacillus GG was inhibited at concentrations of PUFA between 10 and 40 µg/ml (Kankaanpää, Salminen, Isolauri and Lee "The influence of polyunsaturated fatty acids on probiotics growth and adhesion", FEMS Microbiology Letters 194 (2001) 149 - 153).

### Summary of the Invention

The present invention therefore pertains to formulae intended both for infants and young children. The formula of the invention comprises a source of proteins, a source of lipids, a source of carbohydrates and a probiotic wherein the source of lipids includes ARA and DHA and wherein the DHA content is between 0.2 and 0.5% of the total fatty acids in the lipid source.

The invention further provides a method for strengthening natural immune defences of an infant or a young child consisting in fully or partly feeding the infant or child with the said formula.

### Detailed Description of the Invention

In the present specification the following words are given a definition that must be taken into account when reading and interpreting the description, examples and claims.

Infant: according to the Commission Directive 91/321/EEC of 14 May 1991 on infant formulae and follow-on formulae, article 1.2(a), the term "infants" means children under the age of 12 months. This definition is adopted in the present specification.

Young Children: according to the Commission Directive 91/321/EEC of 14 May 1991 on infant formulae and follow-on formulae, article 1.2(b), the term "young children" means children, aged between one and three years. This definition is adopted in the present specification.

Infant formulae: according to the Commission Directive 91/321/EEC of 14 May 1991 on infant formulae and follow-on formulae, article 1.2(c), the term "infant formula" means foodstuffs intended for particular nutritional use by infants during the first four to six months of life and satisfying by themselves the nutritional requirements of this category of persons. This definition is adopted in the present specification. It has to be understood that infants can be fed solely with infant formulas, or that the infant formula can be used by the carer as a complement of human milk. It is synonymous to the widely used expression "starter formula".

Follow-on formulae: according to the Commission Directive 91/321/EEC of 14 May 1991 on infant formulae and follow-on formula, article 1.2(d), the term "follow-on formulae" means foodstuffs intended for particular nutritional use by infants aged over four months and constituting the principal liquid element in a progressively diversified diet of this category of persons. This definition is adopted in the present specification.

Probiotic: according to the paper Probiotics in Man and Animals, J. Appl Bacteriol. 66: 365-378, a probiotic is defined as a live microbial feed supplement which beneficially affects the host animal by improving its intestinal microbial balance.

According to a first aspect of the invention, there is provided a nutritional formula for infants (including a starter composition) or young children. As already mentioned, it is an object of the invention to provide a unique combination of protective nutrients ensuring improved natural defences compared to bottle-fed infants and children.

Human milk contains docosahexaenoic acid (DHA) and arachidonic acid (ARA) and thus breast-feeding provides infants with preformed LC-PUFAs. The DHA content of human milk varies considerably within populations and is strongly influenced by material diet. Globally, the DHA content of milk from mothers consuming Western diets ranges from 0.1 to 0.4%, with a mean of 0.25%, whereas in mothers consuming non-Western diets, the DHA content of milk is greater, ranging from 0.1 to 1.4%, with a mean of 0.6%. However, amounts of 0.2 to 0.3% are generally accepted as representative. The ARA content of human milk is less influenced by the diet than DHA. Globally, the ARA content of human milk from mothers consuming Western diets ranges from 0.2 to 0.7%, with a mean of 0.45%, whereas in mothers consuming non-Western diets, the ARA content ranges from 0.4 to 1.2%, with a mean of 0.6%. Both DHA and ARA levels are influenced by the duration of lactation and tend to decrease from colostrum to transitional and mature milk.

As there is competition between fatty acids of the n-3 and n-6 pathways with respect to elongation and desaturation, as well as for incorporation into phospholipids and conversion to eicosanoids_{;} we have balanced the fat in infant formulae with respect to n-6 and n-3 fatty acids. Supplementation of infants formula with only alpha-linolenic acid as a source of n-3 fatty acids, even in the recommended balance with linoleic acid, does not support DHA status equivalent to that of breast-fed infants. Indeed, numerous studies have demonstrated higher levels of DHA in circulating pools of lipids: plasma phospholipids, red blood cell lipids, red blood cell phospholipids, red blood cell phosphatidylethanolamine in breast-fed compared to formula-fed infants. The arachidonic acid status in most cases is not affected and similar to that of breastfed infants. Numerous studies have shown that it is possible to achieve DHA levels in the various blood pools of formula-fed infants similar to or even higher than those of breastfed infants by supplementing the formula with DHA.

Formulae according to the invention thus comprise DHA. High amounts of DHA alone, or use of DHA sources providing high levels of EPA, a fatty acid precursor of DHA, may however lead to depletion of the arachidonic status. Thus, DHA in formulae according to the present invention is preferably provided by a low EPA fish oil at a level which has been shown to achieve DHA levels in the various blood pools of formula-fed infants similar to those of breast-fed infants. The DHA content is between 0.2 and 0.5% of total fatty acids in the lipid source.

ARA is widely distributed in all cell membranes; it is the major LC-PUFA in most peripheral tissues (e. g. heart, liver) and it is present in larger amounts in nervous tissue. It is also the precursor of biological substances known collectively as eicosanoids: prostaglandins, leukotrienes and thromboxanes which have a role in immunoregulation, in inflammatory processes and muscle contraption. Arachidonic acid is considered as being important for optimal growth, as a significant correlation has been found between plasma arachidonic acid levels and infant body growth. Thus, the lipid source of formulae of the present invention also includes a source of ARA which may be from a fungal source such as Mortierella Alpina. The ratio of ARA:DHA is preferably between 0.8:1 and 1.2:1, more preferably 1:1.

In contrast to ARA, DHA accounts only for a small percentage of the fatty acid content in most tissues, except in neuronal tissues, such as the retina and the brain. In the retina, it is concentrated in the specialized membranes of the photoreceptor outer segments that are dynamic structures whose components are renewed daily, and represents up to 50% of the fatty acids of the main phospholipids. Animals with low DHA retinal levels present with abnormal electroretinograms. In the brain, the total amount of DHA increases dramatically during the brain growth spurt, both because of the growth of brain in size (from 100 g at the beginning of the third trimester of pregnancy to about 1100 g 18 months postnatally), but also because there is an increase in the relative DHA content, which has been calculated to increase approximately 35 mg per week from the beginning of the last trimester of pregnancy till the end of the first year of life.

Preferably, the remainder of the fats in the lipid source according to the invention are carefully selected as will now be described. Fat provides about half of the dietary energy and constitutes the major energy stores in the bodies of infants and young children. Presently, there is growing interest in the quality of the dietary lipid supply during infancy as a major determinant of growth, visual and neural development, and long-term health. Thus, the selection of the dietary lipid supply during early life is considered to be of great importance.

Because of the small size of their stomach and their limited tolerance of hypertonic foods, infants require a concentrated source of energy. Of the 3 nutrients supplying energy, fat provides 9 kcal per gram, i.e. more than twice the energy present in carbohydrates or proteins. Most experts recommend that in infant and follow-on formulae fat should supply from 30% to 55% of the total energy. Preferably, the fats used in the formulae of the invention are predominantly vegetable fats. However, they and skim milk naturally contain traces of milk fat, and so a very small percentage of milk fat is likely to be present.

Fatty acid composition of the diet determines fatty acid composition of all tissues, including storage tissues. The fat blend used in the formulae of the invention therefore preferably has an overall fatty acid composition as close as possible to that of human milk, in order to ensure similar membrane plasticity and same mobilization of energy in case of increased needs. Thus, the preferred fat blend supplies the essential fatty acids (linoleic and α-linolenic acids), as well as adequate quantities of oleic acid, palmitic acid, lauric acid and myristic acid.

As previously mentioned, formulae according to the invention comprise at least one probiotic, in order to offer all infants, whatever their mode of delivery or their hygienic environment, the advantages of a protective intestinal flora.

Preferred probiotics are those which as a whole are safe, are L (+) lactic acid producing cultures and have acceptable shelf-life for products such as infant and follow-on formulae which are required to remain stable and effective for up to 36 months. Examples of preferred probiotics are:-
*Bifidobacterium lactis,* first sold by Christian Hansen company;
*Streptococcus thermophilus* provided under the name TH4 by Chr. Hansen, Denmark;
*Lactobacillus paracasei rhamnosus* GG (ATCC 53103) provided by Valio Oy, Finland;
*Bifidobacterium longum BB536* provided by Morinaga Milk Industry Co. Ltd, Japan.

The probiotics according to the present aspect of the invention are preferably present in an amount of 10⁶ to 10⁹cfu/ grams of dry product, preferably 10⁶ to 10⁸ cfu/g, and even more preferably 2*10⁷ cfu/grams of dry product.

The composition according to the present invention comprises at least one probiotic strain but combinations of different strains may also be used, particularly in follow-on formulae. If such a combination is to be used, it will preferably include at least one Bifidobacteria and at least one Lactobacillus. A particularly preferred combination is Bifidobacterium longum BB536 and Lactobacillus paracasei rhamnosus GG.

Dietary protein provides the essential amino acids necessary for protein synthesis and growth and protein quality is as important as protein quantity. Until recently, it was thought that in order to supply enough of the essential amino acids, formulae based on cows' milk needed a protein content significantly higher than that of the reference human milk. The protein content of regular whey-adapted formulae ranges from 2.1 to 2.6 g per 100 kcal, whereas the content of human milk ranges from 1.4 to 1.8 g per 100 kcal. Excess protein intake may induce metabolic stress on infant organs that have not fully developed. Following paediatric recommendations for lowering protein density of infant formulae, clinical trials in infants fed formulae containing protein densities between 1.6 and 2.0 g / 100 kcal have been reported. However, these attempts to lower protein content in a formula using traditional cow's milk protein sources or mixing the currently available fractions - casein and whey -, although demonstrating the principle was conceivable, failed to reproduce all the indices of human milk protein metabolism or to ensure the satisfactory growth of infants. For instance, results have shown a global plasma amino acid pattern different to that of breast-fed infants, depressed plasma tryptophan levels, elevated plasma threonine levels, delay in growth, and higher energy intake suggesting an increased fat deposition which may be responsible for obesity in later life.

The protein fraction in cows' milk is a mixture of several proteins, which all have a different amino acid profile. Caseino-glyco-macropeptide (CGMP) is a protein that is found in this fraction. It comes from the kappa-casein that is split up by proteolytic cleavage into 2/3 para-kappa-casein, an insoluble fraction that remains in the casein fraction and 1/3 CGMP, a soluble fraction that is found in the whey fraction. An original fractionation process of whey proteins has been developed and is explained in EP 880902; this process allows the removal of practically all the CGMP (a fraction rich in threonine and poor in tryptophan) from bovine whey thereby increasing the alpha-lactalbumin proportion (a fraction very rich in tryptophan). By combining this modified sweet whey (MSW) fraction with skim milk, and with the addition of some free L-histidine and L-arginine (in order to reach the minimum amounts of these amino acids required by EC Directive), the protein source of the formula according to the invention has an amino acid profile much closer to that of human milk, characterised in particular by comparable tryptophan and threonine levels, allowing the adaptation of its protein content to that of human milk.

The nutritional value of this protein mixture has been measured in rats. The results show (see table 1) that this formulation has a Protein Efficiency Ratio (PER), a nitrogen digestibility, a Biological Value (BV), and a Net Protein Utilisation (NPU) comparable to standard whey-adapted formulae.

**Table 1**

| **Nutritional parameters** | **Casein** | **standard whey-adapted formula** | **formulation of the invention** |
|---|---|---|---|
| PER | 1.36 | 2.49 | 2.70 |
| Relative PER (casein = 100%) | 100.0 | 182.8 | 198.3 |
| Digestibility (%) | 96.7 | 92.8 | 91.4 |
| BV | 0.88 | 0.96 | 0.96 |
| NPU (%) | 85.4 | 88.8 | 87.5 |

Moreover, rats fed on the formula according to the invention showed significant lower plasma threonine levels and increased plasma tryptophan levels, compared to rats fed on standard whey-adapted formulae.

The protein content of formulae according to the present invention is preferably no more than 2g/100 kcal, more preferably less than 1.85, most preferably between 1.8 and 1.85 g/100 kcal. This level is in line with recent data assessing protein requirements during early life, which has shown that recommendations for optimal protein intakes are lower than they have been reported in the past.

To ensure optimal protein synthesis, and therefore optimal growth, essential and semi-essential (i.e. essential only during infancy) amino acids need to be supplied in the same quantities as in human milk. Formulae according to the invention are preferably either whey enriched (casein / whey ratio set around 40/60) or, more preferably, whey predominant (casein / whey ratio preferably set at 30/70 or even more, such as 20/80). A preferred amino acid profile for formulae according to the invention is comparable to that of human milk (see table 2).

**Table 2**

| **Amino acid (g /16 g N)** | **Human milk** | | | **invention (representative values)** |
|---|---|---|---|---|
| | **mean** | **lowest value** | **highest value** | |
| Isoleucine * | 6.4 | 5.7 | 6.8 | 5.8 |
| Leucine * | 11.5 | 11.0 | 11.9 | 11.9 |
| Lysine * | 7.9 | 7.4 | 8.4 | 10.0 |
| Methionine * | 1.7 | 1.3 | 2.1 | 2.5 |
| Cystine** | 2.3 | 1.7 | 2.9 | 2.4 |
| Phenylalanine* | 4.6 | 4.2 | 5.1 | 4.6 |
| Tyrosine** | 4.7 | 3.3 | 6.3 | 4.0 |
| Threonine * | 5.6 | 5.3 | 6.6 | 5.4 |
| Tryptophan * | 2.3 | 1.8 | 2.6 | 2.1 |
| Valine * | 6.8 | 5.9 | 8.0 | 5.9 |
| Arginine ** | 4.2 | 3.5 | 4.9 | 4.5 |
| Histidine ** | 2.8 | 2.4 | 3.8 | 2.5 |
| Alanine | 4.8 | 4.5 | 5.3 | 5.1 |
| Aspartic acid | 10.4 | 10.1 | 10.8 | 11.1 |
| Glutamic acid | 19.6 | 17.6 | 22.7 | 19.7 |
| Glycine | 3.2 | 2.8 | 3.6 | 2.7 |
| Proline | 10.2 | 8.9 | 11.2 | 7.8 |
| Serine | 5.6 | 5.0 | 5.9 | 5.3 |

| | | | | |
|---|---|---|---|---|
| All values corrected to 40% NH₃ * essential amino acids ** semi-essential amino acids | | | | |

The proteins may be either intact or partially hydrolysed by a process such as that described in European Patent No 322589.

Preferably, the sole source of carbohydrates of the composition according to the present invention is lactose. Carbohydrates constitute an important source of energy in the diet of the newborn infant. Lactose is the natural carbohydrate in human milk. Most infants in good health can digest lactose adequately. Further, lactose is associated with stool acidity and the development of a Bifidobacteria and lactobacilli preponderant microflora in the large intestine similar to that of breastfed babies. This is thought to be important in suppressing the growth of undesirable bacteria in the large intestine. Moreover, lactose has been shown to enhance absorption and retention of calcium and probably other minerals. In a recent study, it has been shown that calcium absorption is 10% greater from a lactose-containing formula compared with the same formula in which the lactose was replaced by glucose polymers.

The formulae according to the invention may also supply semi-essential nutrients which may be needed in particular conditions (e. g. taurine, nucleotides, carnitine, selenium).

Taurine is a free amino acid, which is not used to build up protein molecules. It has been shown to be involved in many physiological functions, e.g., as a trophic factor in the development of the central nervous system, maintaining the structural integrity of the membrane, regulating calcium homeostasis, as an osmolyte, a neuromodulator, and a neurotransmitter. It also conjugates with bile acids to form bile salts (essential for micelle formation and fat absorption).

Nucleotides are non protein nitrogen compounds which contain three characteristic components: a nitrogenous base, a sugar (ribose or deoxy-ribose), and one or more phosphate groups. Total nucleotide content in human milk represents 2 to 5% of the non-protein nitrogen. Cow's milk contains lower concentrations of nucleotides than human milk and its nucleotide profile differs markedly from that of human milk. Addition of nucleotides in the present infant formula follows the physiological pattern of nucleotides levels in human milk, with a predominance of easily metabolised pyrimidines over less desirable purines: addition of nucleotides to the infant formula is safe. The levels of addition are within the range allowed by the European Union Scientific Committee for Food and the European Directive.

Carnitine is a particular nitrogenous compound, which belongs to a group of food factors known as vitamin-like nutrients. It performs a crucial role in the energy supply of tissues during foetal life and in the neonatal period by facilitating the transport of long chain fatty acids into the mitochondria where beta-oxidation occurs. Fatty acids are indeed not able to pass in free form through the mitochondrial wall; the transfer into the mitochondria is governed by at least three enzymatic systems, namely carnitine - palmitoyl transferases I and II and carnitine -translocase, in which carnitine participates. Thus, carnitine is required for proper lipid oxidation and carnitine deficiency or low carnitine intake can lead to impaired fat utilisation and altered lipid metabolism. Carnitine has also a role in other metabolic processes, such as ketogenesis, lypolysis, and the maintenance of thermogenesis and nitrogen metabolism. Moreover, carnitine has been shown to improve utilisation of medium chain triglycerides in infants. Newborns have relatively low carnitine reserves and a very low activity of the enzyme catalysing the last step in the carnitine synthesis. Thus newborns are particularly at risk of becoming carnitine-deficient in the absence of an adequate supply of exogenous carnitine. Carnitine is preferably added to infant formulae, in order to reach a level close to that of human milk.

Formulae according to the invention may be in powder form or a ready to drink liquid. In the case of a powder formula, the following feeding table (table 4) may be used as a guide. However, the quantities may be changed according to medical advice. The introduction of an infant formula should be carried out under medical supervision. The standard reconstitution of formulae according to the invention is 12.9%, i.e. 12.9 g powder for 90 mL of water, which gives a caloric density of 67 kcal/100mL.

**Table 4**

| | quantity per feed | | No. of feeds per day | |
|---|---|---|---|---|
| Age of infant | Previously boiled water (mL) | number. of measuring scoops | Formula | Others |
| 1^{st} and 2^{nd} weeks | 90 | 3 | 6 | - |
| 3^{rd} and 4^{th} weeks | 120 | 4 | 5 | - |
| 2^{nd} month | 150 | 5 | 5 | - |
| 3^{rd} and 4^{th} months | 180 | 6 | 5 | - |
| 5^{th} and 6^{th} months | 210 | 7 | 5 | - |
| from the 7^{th} month onwards | 210 | 7 | 4-3 | 1-2 |

In the case of a ready-to-drink liquid, special care needs to be taken to ensure that the probiotic does not accidentally come into contact with the liquid. Preferably, the probiotic is stored in powder form separate from the liquid, and is incorporated and homogenised into the liquid just before consumption, e.g. up to two hours before consumption.

The present invention also relates to a method of strengthening natural immune defences of an infant or a young child consisting in fully or partly feeding the infant or child with a formula according to the invention

Intestinal mucosa is one important location for the immune system and the gastro-intestinal microflora plays a dominant role in the development of the gut-associated lymphoid tissue (GALT). This highly organized immune system consists of lymphoid follicles that can be either isolated or grouped in Peyer's patches present in the deep part of the mucosa and the submucosa of the small intestine. GALT has the capacity to discriminate between pathogenic micro organisms to which it responds dynamically, and the vast array of dietary antigens and commensal microbial flora to which it remains tolerant. Probiotics interact with the immune system at many levels, including cytokine production, mononuclear cells proliferation, macrophage phagocytosis and killing, modulation of autoimmunity, and immunity to bacterial and protozoan pathogens.
These immunological properties may be strain-specific. *Bifidobacterium lactis* has been shown to positively influence mucosal immunity: in adult subjects, *B. lactis* enhances stimulation of phagocytosis by peripheral blood lymphocytes whereas in infants, *B*. *lactis* enhances secretion of faecal IgA , immunoglobulins which play an important role in pathogens elimination.
More important, this immune stimulation results in a clear health benefit, i.e. reduction of the risk of diarrhoea in infants at high risk of contamination as hospital environment and in the more usual conditions of day-care centres. A similar trend was found recently in a study comparing a starter whey hydrolysed formula with different protein levels and *B. lactis* addition. Salivary rotavirus-specific IgA titres are a good indicator of rotavirus infections. Whereas they are not detected in healthy neonates, they are increased in infected infants. Infants and children fed a *B. lactis* enriched formula have less often an increase in their salivary anti-rotavirus titres when exposed to a contaminated environment, supporting the hypothesis that *B. lactis* supplementation protects against rotavirus infection.
Inflammation (usually characterised by redness, swelling, heat and pain) is a normal, immediate response of the body to infection. It is part of the normal, innate immune system. A too strong immune reaction may thus lead to excessive inflammatory reaction. Allergy is also the result of an exacerbated immune reaction due to inappropriate recognition and response to antigens. Appropriate stimulation of the immune system should therefore result in adequate protective mucosal immunity without excessive inflammation and develop systemic oral tolerance.
In the newborn, the pattern of immune response is skewed towards Th-2 type of response, leading to allergic reactions, and will evolve during postnatal maturation towards a balanced Th-1/Th-2 response.
The intestinal flora counterbalances Th2 activity and affects the development of many other immune parameters. Differences in intestinal flora composition exist between infants developing allergy and healthy infants: infants with atopic dermatitis are less frequently colonized by bifidobacteria as compared to healthy ones Probiotics are therefore considered as potential modulators of the allergic reactions. But similarly to the immune protection, this activity is strain-specific. The anti-inflammatory properties of *B. lactis* have been shown first in *in vitro* models of cell cultures and confirmed in highly sensitised infants who did not react to feeding with an extensively hydrolysed infant formula. In such infants, *B. lactis* reduces the symptoms of atopic dermatitis. Further, supplementation with *B. lactis* prevents the increase in the numbers of bacteroides and *E*. *coli* during weaning, and high numbers of bacteroides and *E*. *coli* are associated with the extent of atopic sensitisation in infants with atopic eczema.
The so-called "hygiene hypothesis" suggests that the increase in allergic disease may be due to a lack of stimulation of the immune system by microbial exposure and resulting prolongation of the immature neonatal pattern of immune response well into the first years of life. Since the pattern of response associated with the first encounter with an antigen is likely to be programmed into long-term immunological memory, an innocuous early life exposure as realized by selected probiotics such as B. lactis may further contribute to an optimal health status later in life.

The quantity and quality of dietary lipids and their metabolism are of major importance for the growth, body composition, development and long-term health of children, both in health and disease. Lipids are the major source of energy in early childhood and supply essential lipid-soluble vitamins and polyunsaturated fatty acids that are required in relatively high amounts during early growth. Lipids affect the composition of membrane structures, and modulate membrane functions as well as the functional development of the central nervous system. Some LC-PUFAs serve as precursors for bioactive lipid mediators, including prostaglandins, thromboxanes and leukotrienes, which are powerful regulators of numerous cell functions such as thrombocyte aggregation, inflammatory reactions and immune functions.

The fatty acid composition of inflammatory and immune cells is sensitive to change according to the fatty acid composition of the diet. In particular, the proportion of different types of PUFAs in these cells is readily changed, and this provides a link between dietary PUFA intake, inflammation, and immunity. The n-6 PUFA ARA is the precursor of prostaglandins, leukotrienes, and related compounds, which have important roles in inflammation and in the regulation of immunity. Among other compounds, lipids, especially n-3 polyunsaturated fatty acids, were shown to influence the immune response. Fish oil contains the n-3 PUFAs EPA and DHA. Feeding fish oil results in partial replacement of ARA in cell membranes by EPA. This leads to decreased production of ARA-derived mediators. In addition, EPA is a substrate for cyclooxygenase and lipoxygenase and gives rise to mediators that often have different biological actions or potencies than those formed from ARA. Animal studies have shown that dietary fish oil results in altered lymphocyte function and in suppressed production of pro-inflammatory cytokines by macrophages. Supplementation of the diet of healthy human volunteers with fish oil-derived n-3 PUFA results in decreased monocyte and neutrophil chemotaxis and decreased production of pro-inflammatory cytokines.
Cyclooxygenase and lipoxygenase catalyse the synthesis of eicosanoids from LCPUFA precursors (ARA, EPA). Eicosanoids are C-20 PUFA derivatives that include prostaglandins, thromboxanes, and leukotrienes. Among their ubiquitous biological effects such as on the immune cell functions, they are involved in the normal regulation of gastric secretion and gastric motility, as well as in gastric mucosal defence. n-3 Fatty acids influence inflammatory cell activation processes from signal transduction to protein expression even involving effects at the genomic level. n-3 Fatty acid-mediated mechanisms decreased cytokine-induced adhesion molecule expression, thereby reducing inflammatory leucocyte-endothelium interactions and modified lipid mediator synthesis, thus influencing the trans-endothelial migration of leucocytes and leucocyte trafficking in general. Even the metabolic repertoire of specific immunocompetent cells such as cytokine release or proliferation is modified by n-3 fatty acids.
PUFAs have implications on T-cell function for the neonates. Infant survival depends on the ability to respond effectively and appropriately to environmental challenges. Infants are born with a degree of immunological immaturity that renders them susceptible to infection and abnormal dietary responses (allergies). T-lymphocyte function is poorly developed at birth. The reduced ability of infants to respond to mitogens may be the result of the low number of CD45RO+ (memory/antigen-primed) T cells or their limited ability to produce cytokines, particularly interferon-γ and interleukins IL-4, and IL-10. There have been many important changes in optimising breast milk substitutes for infants; however, few have been directed at replacing factors in breast milk that convey immune benefits.

Compared with standard formula, feeding a formula containing DHA and ARA increases the proportion of antigen mature (CD45RO+) CD4+ cells, improves IL-10 production, and reduces IL-2 production to levels not different from those of human milk-fed infants.
After the oral mucosa, the intestinal epithelium and its associated gut-associated lymphoid tissue are the primary targets of dietary components.
Plasma membranes of many cell types contain domains enriched in specific lipids (saturated fatty acids, sphingolipids) and cholesterol, called lipid rafts. These serve as entry sites for several receptor-mediated signalling events by stabilising receptor/kinase interactions, suggesting an involvement in the initiation of signalling cascades. Cross-linking of surface receptors in hematopoietic cells results in the enrichment of these receptors in the rafts along with other downstream signalling molecules. A possible explanation of how signal is transduced through the plasma membrane has arisen from the concept of rafts. From the study of cellular responses in the plasma membrane which enrich members of the Src-family tyrosine kinase, rafts can function as centres of signal transduction by forming patches. Under physiological conditions, these elements synergise to successfully transduce a signal at the plasma membrane.

In T lymphocytes, key T cell antigen receptor (TCR) signalling molecules associate with rafts, disrupting raft-association of certain of these abrogates TCR signalling. The TCR itself associates with lipid rafts, and TCR cross-linking causes aggregation of raft-associated proteins. Furthermore, raft aggregation promotes tyrosine phosphorylation and recruitment of signalling proteins, but excludes the tyrosine phosphatase CD45. Rafts are thus suggested to be important in controlling appropriate protein interactions in hematopoietic cells, and aggregation of rafts following receptor ligation may be a general mechanism for promoting immune cell signalling. Although not wishing to be bound by theory, we believe that the rafts, rich in saturated fatty acids, are influenced by dietary LC-PUFAs explaining part of their biological effects on immune function.
A clear effect of LC-PUFAs or their precursors have been demonstrated on functions such as systemic immunity or lipid and carbohydrate metabolism, although most of them have been done on adult humans or animals.

Dietary LC-PUFAs are absorbed and incorporated to the membranes of the enterocytes. They appear to modulate the local inflammatory response and promote intestinal repair after stress. Therefore, dietary LC-PUFAs improve the repair of small intestine, for example in individuals that have been previously malnourished. The possible mechanisms whereby LC-PUFAs can affect the inflammatory cascade are multiple. Nonetheless, the role of LC-PUFAs in specifically modulating gut inflammation remains unclear.

Several reports suggest up-regulation of the non-specific barrier function by the products of PUFAs. Thus eicosanoids, particularly those derived from ARA, would affect intestinal secretion, mucus secretion and density of surfactant in mucus, phospholipid synthesis and provide cytoprotection to the GI mucosa. It has also been suggested that intestinal glycosyltransferases are modulated by the global unsaturation index of the fatty acids in the diet, while occludin (major component of the tight junction complex) expression would be up-regulated by gamma-linolenic acid (18:3n-6) and eicosapentaenoic acids and down-regulated by AA and linoleic acid (18:2n-6).

Finally, PUFAs and LC-PUFAs might be able to modulate the composition of the intestinal flora. Linoleic and alpha-linolenic acids suppress the proliferation of *Staphylococcus aureus.* Similarly, relatively high concentrations, although still in the physiological range, of free linoleic, gamma-linolenic, arachidonic, alpha-linolenic, and docosahexaenoic acids inhibit growth and mucus adhesion of *Lactobacillus GG, casei* and *bulgaricus*. Moreover, milder concentrations of gamma-linolenic acid and ARA promote growth and mucus adhesion of *L. casei.*
Furthermore, the adhesion of those bacteria to Caco-2 cells grown in PUFA-containing media is modulated by the type and concentration of LG-PUFAs. Given that the ability to adhere to the intestinal surfaces appears to be important for the functionality of the probiotics and for the virulence of the pathogenic bacteria, we believe that the supplementation with LC-PUFAs affects the efficacy of the probiotic and the invasive capacity of the pathogens.

Although not wishing to be bound by theory, we believe that the beneficial effect of probiotics are improved by their combination with LC-PUFAs, and that LC-PUFAs promote the actions of probiotics. Therefore, a formula according to the invention exploits the synergic effect of these two components.

### Examples

The following examples are illustrative of some of the products and methods of making the same falling within the scope of the present invention. They are not to be considered in any way limitative of the invention. Changes and modifications can be made with respect to the invention. That is, the skilled person will recognise many variations in these examples to cover a wide range of formulas, ingredients, processing, and mixtures to rationally adjust the naturally occurring levels of the compounds of the invention for a variety of applications.

### Example 1

The following example of a preferred formula according to the invention is illustrative only

| Nutrient | | per 100kcal | per litre |
|---|---|---|---|
| Energy (kcal) | | 100 | 670 |
| Protein (g) | | 1.83 | 12.3 |
| Total Fat (g) | | 5.3 | 35.7 |
| of which:- | | | |
| | Linoleic acid (g) | 0.79 | 5.3 |
| | α-Linolenic acid (mg) | 101 | 675 |
| | DHA (mg) | 12 | 77 |
| | ARA (mg) | 12 | 77 |
| Lactose (g) | | 11.2 | 74.7 |
| Minerals (g) | | 0.37 | 2.5 |
| Na (mg) | | 23 | 150 |
| K (mg) | | 89 | 590 |
| Cl (mg) | | 64 | 430 |
| Ca (mg) | | 62 | 410 |
| P (mg) | | 31 | 210 |
| Mg (mg) | | 7 | 50 |
| Mn (µg) | | 8 | 50 |
| Se (µg) | | 2 | 13 |
| Vitamin A (µg RE) | | 105 | 700 |
| Vitamin D (µg) | | 1.5 | 10 |
| Vitamin E (mg TE) | | 0.8 | 5.4 |
| Vitamin K1 (µg) | | 8 | 54 |
| Vitamin C (mg) | | 10 | 67 |
| Vitamin B1 (mg) | | 0.07 | 0.47 |
| Vitamin B2 (mg) | | 0.15 | 1.0 |
| Niacin (mg) | | 1 | 6.7 |
| Vitamin B6 (mg) | | 0.075 | 0.50 |
| Folic acid (µg) | | 9 | 60 |
| Pantothenic acid (mg) | | 0.45 | 3 |
| Vitamin B12 (µg) | | 0.3 | 2 |
| Biotin (µg) | | 2.2 | 15 |
| Choline (mg) | | 10 | 67 |
| Fe (mg) | | 1.2 | 8 |
| I (µg) | | 15 | 100 |
| Cu (mg) | | 0.06 | 0.4 |
| Zn (mg) | | 0.75 | 5 |

| | | | |
|---|---|---|---|
| Bifidobacterium longum BB 536: 1 x 10⁷ cfu/gram of dry product Lactobacillus paracasei rhamnosus GG: 2 x 10⁷ cfu/gram of dry product | | | |

### Example 2

Over 300 healthy infants of birth weight at least 2,500g whose mothers have elected not to breast feed after the 5^{th} day of their life and who are participating in the vaccination programmes for hepatitis B, poliomyelitis, diptheria, tetanus and pertussis are enrolled in a randomised, controlled, double blind, single centre clinical trial of two groups in parallel. The first group is fed the formula of Example 1 and the second group is fed a similar formula but without probiotics. The infants' response to the vaccination programmes is assessed using IgG titres taken as follows:-

| | |
|---|---|
| Hepatitis B | 7 months and 11 months |
| Poliomyelitis | 6 months and 12 months |
| DTP | 6 months and 12 months |

Also, anti bovine β-lactoglobulin IgG and IgE titres are taken at 2 and 4 months and determination of bacterial colonisation is assessed by means of stool analysis for total Lactobacillae, total Bifidobacteria, Clostridium perfringens, Enterobacteriaceae, Bacteroides and probiotics at 2, 4 and 12 months. Finally, an assessment of gut barrier function as evidenced by IgA and caloprotectin levels in stools is made at 2 and 4 months.

In addition, anthropometric measurements (weight gain, length an head circumference) usual in a study of this type are carried out at recruitment and each month thereafter and, at the same time these measurements are taken, observations are made of digestive tolerance (stool characteristics, incidence of vomiting and regurgitation, frequency and duration of colic) and frequency of episodes of morbidity are noted (number of times seen by healthcare professionals plus episodes of ill health).

It is found that infants fed the formula of the invention generally display strengthened immune defences as demonstrated by an enhanced response to vaccinations and/or improved gut barrier function and lower levels of intolerance of cows' milk protein coupled with satisfactory physical development) when compared with the control group.

## Claims

1. Infant or follow-on formula comprising a source of proteins, a source of lipids, a source of carbohydrates and a probiotic wherein the source of lipids includes ARA and DHA and the DHA content is between 0.2 and 0.5% of total fatty acids in the lipid source.

2. Formula according to claim 1 wherein the ratio of ARA:DHA is between 0.8:1 and 1.2:1, preferably 1:1.

3. Formula according to any preceding claim, wherein the probiotic is a Bifidobacteria or a Lactobacillus.

4. Formula according to claim 3 wherein the Bifidobacteria is Bifidobacterium longum BB 536.

5. Formula according to claim 3 wherein the Lactobacillus is Lactobacillus paracasei rhamnosus GG.

6. Formula according to any preceding claim which contains both a Bifidobacterium and a Lactobacillus.

7. Formula according to claim 6 wherein the Bifidobacteria is Bifidobacterium longum BB 536 and the Lactobacillus is Lactobacillus paracasei rhamnosus GG.

8. Formula according to any preceding claim wherein at least 40% of the proteins are modified sweet whey proteins with no CGMP or reduced CGMP.

9. Formula according to claim 8 wherein the wherein at least 60% of the proteins are modified sweet whey proteins comprising no CGMP or reduced CGMP.

10. Formula according to claim 8 or claim 9 wherein the proteins are present in a maximum proportion of 2g/100 kcal, preferably 1.85, most preferably between 1.8 and 1.85 g/100 kcal.

11. The use of a probiotic and a source of lipids including DHA and ARA in the manufacture of a composition comprising a source of proteins, a source of lipids, and a source of carbohydrates for strengthening natural immune defences of an infant or a baby by fully or partly feeding said infant or baby with said formula wherein the DHA content is between 0.2 and 0.5% of total fatty acids in the lipid source.

## Patentansprüche

1. Säuglingsanfangs- oder Folgenahrung mit einer Quelle von Proteinen, einer Quelle von Lipiden, einer Quelle von Kohlenhydraten und einem Probiotikum, wobei die Quelle von Lipiden ARA und DHA einschließt und der DHA-Gehalt zwischen 0,2 und 0,5% der gesamten Fettsäuren in der Lipidquelle beträgt.

2. Nahrung nach Anspruch 1, wobei das Verhältnis von ARA:DHA zwischen 0,8:1 und 1,2:1, vorzugsweise 1:1, beträgt.

3. Nahrung nach irgendeinem vorausgehenden Anspruch, wobei das Probiotikum ein Bifidobakterium oder ein Lactobacillus ist.

4. Nahrung nach Anspruch 3, wobei das Bifidobakterium Bifidobacterium longum BB 536 ist.

5. Nahrung nach Anspruch 3, wobei der Lactobacillus Lactobacillus paracasei rhamnosus GG ist.

6. Nahrung nach irgendeinem vorausgehenden Anspruch, die sowohl ein Bifidobakterium als auch einen Lactobacillus enthält.

7. Nahrung nach Anspruch 6, wobei das Bifidobakterium Bifidobacterium longum BB 536 ist und der Lactobacillus Lactobacillus paracasei rhamnosus GG ist.

8. Nahrung nach irgendeinem vorausgehenden Anspruch, wobei wenigstens 40% der Proteine modifizierte Süßmolkenproteine ohne CGMP oder mit vermindertem CGMP sind.

9. Nahrung nach Anspruch 8, wobei wenigstens 60% der Proteine modifizierte Süßmolkenproteine sind, die kein CGMP oder ein vermindertes CGMP umfassen.

10. Nahrung nach Anspruch 8 oder Anspruch 9, wobei die Proteine in einem maximalen Anteil von 2 g/100 kcal, vorzugsweise von 1,85, am stärksten bevorzugt zwischen 1,8 und 1,85 g/100 kcal vorhanden sind.

11. Verwendung eines Probiotikums und einer Lipidquelle, die DHA und ARA einschließt, bei der Herstellung einer Zusammensetzung, die eine Quelle von Proteinen, eine Quelle von Lipiden und eine Quelle von Kohlenhydraten umfasst, zur Stärke der natürlichen Immunabwehr eines Kleinkinds oder eines Babies **dadurch**, dass man das Kleinkind oder Baby vollständig oder teilweise mit der genannten Nahrung füttert, wobei der DHA-Gehalt zwischen 0,2 und 0,5% der gesamten Fettsäuren in der Lipidquelle beträgt.

## Revendications

1. Formulation alimentaire pour nourrissons ou formulation d'entretien comprenant une source de protéines, une source de lipides, une source de glucides et un probiotique, dans laquelle la source de lipides comprend les ARA et DHA, la teneur en DHA étant comprise entre 0,2 et 0,5 % des acides gras totaux dans la source de lipides.

2. Formulation suivant la revendication 1, dans laquelle le rapport ARA:DHA est compris entre 0,8:1 et 1,2:1, et est de préférence égal à 1:1.

3. Formulation suivant l'une quelconque des revendications précédentes dans laquelle le probiotique est une bifidobactérie ou un Lactobacillus.

4. Formulation suivant la revendication 3, dans laquelle la bifidobactérie est Bifidobacterium longum BB 536.

5. Formulation suivant la revendication 3, dans laquelle le Lactobacillus est Lactobacillus paracasei rhamnosus GG.

6. Formulation suivant l'une quelconque des revendications précédentes, qui contient à la fois un Bifidobacterium et un Lactobacillus.

7. Formulation suivant la revendication 6, dans laquelle la bifidobactérie est Bifidobacterium longum BB 536 et le Lactobacillus est le Lactobacillus paracasei rhamnosus GG.

8. Formulation suivant l'une quelconque des revendications précédentes dans laquelle au moins 40 % des protéines consistent en protéines de lactosérum doux modifiées à teneur nulle en CGMP ou à teneur réduite en CGMP.

9. Formulation suivant la revendication 8, dans laquelle au moins 60 % des protéines consistent en protéines de lactosérum doux modifiées à teneur nulle en CGMP ou à teneur réduite en CGMP.

10. Formulation suivant la revendication 8 ou la revendication 9, dans laquelle les protéines sont présentes en une proportion maximale de 2 g/100 kcal, avantageusement de 1,85, de préférence de 1,8 à 1,85 g/100 kcal.

11. Utilisation d'un probiotique et d'une source de lipides comprenant DHA et ARA dans la production d'une composition comprenant une source de protéines, une source de lipides et une source de glucides pour le renforcement des défenses immunitaires naturelles d'un nourrisson ou d'un bébé en nourrissant totalement ou partiellement ledit nourrisson ou bébé avec ladite formulation, dans laquelle la teneur en DHA est comprise entre 0,2 et 0,5 % des acides gras totaux dans la source de lipides.
